# EUROPEAN PATENT SPECIFICATION

(11) **EP 2 027 783 B1**
(45) Date of publication and mention of the grant of the patent: **22.06.2016**
(21) Application number: 07730462.4
(22) Date of filing: 13.04.2007
(51) Int. Cl.: A23C 9/13, A61K 36/00, A21D 2/36, A23C 9/152, A61K 31/05, A61K 31/7048, A61K 36/63, A61K 36/752, A23L 2/52, A23L 33/105

(54) **FUNCTIONAL FOOD COMPOSITION THAT IS RICH IN PHENOLIC COMPOUNDS AND USE OF SAID COMPOSITION**
FUNCTIONAL FOOD-ZUSAMMENSETZUNG MIT HOHEM GEHALT AN PHENOLVERBINDUNGEN, UND VERWENDUNG DER ZUSAMMENSETZUNG
COMPOSITION ALIMENTAIRE FONCTIONNELLE RICHE EN COMPOSÉS PHÉNOLIQUES ET UTILISATION DE CETTE COMPOSITION

(30) Priority: 05.06.2006 ES 200601505
(43) Date of publication of application: 25.02.2009
(73) Proprietor: Furfural Espanol, S.A., 30820 Alcantarilla (ES)
(72) Inventor: BENAVENTE-GARCÍA GARCÍA, Obdulio, E-30100 Murcia (ES); CASTILLO SÁNCHEZ, Julián, E-30100 Murcia (ES); LORENTE SALINAS, Juan, E-30002 Murcia (ES)
(74) Representative: Carvajal y Urquijo, Isabel
(86) International application number: PCT/ES2007/000222
(87) International publication number: WO 2007/141352

(56) References cited:
- WO-A1-00/23073
- WO-A1-97/32947
- WO-A2-03/004040
- ANALYST THE: 'Sample preparation in the determination of phenolic compounds in druits' PUBLISHED BY THE ROYAL SOCIETY OF CHEMISTRY, [Online] 2000, pages 1 - 26, XP008091061 Retrieved from the Internet: <URL:http://www.rsc.org/ej/AN/2000/b000080i >
- TRIPOLI E. ET AL.: 'The phenolic compounds of olive oil: structure, biological activity and beneficial effects on human health' NUTRITION RESEARCH REVIEWS vol. 18, no. 1, 2005, pages 98 - 112, XP009070306
- DEL RIO J.A. ET AL.: 'Involvement of phenolic compounds in the antifungal defense mechanisms of Olea europaea L. and Citrus sp' RECENT RESEARCH DEVELOPMENTS INA AGRICULTURAL & FOOD CHEMISTRY vol. 4, no. 2, 2000, pages 331 - 341, XP008091886

## Description

### Object of the Invention

The present invention relates to a functional food composition rich in phenolic compounds and to the use thereof in the preparation of functional food products and dietary supplements for preventing, reducing or treating the problems caused by excess blood cholesterol and triglycerides.

### Background of the Invention

The current knowledge about the origin of atherosclerosis is that it is an inflammatory process involving an acute response phase. Inflammatory processes are a natural biological response to homeostatis alterations such as infections, tissue lesions, neoplasias or immunity problems, for example. The activation of the acute inflammation phase is marked by the release of interleukin-6, triggering the production of certain proteins (fibrinogen, C-reactive protein (CRP), amyloid A protein) leading to the inflammatory reaction. The tissues by themselves contain high levels of cytokines and proteins responsible for acute inflammation, which occasionally results in a localized inflammatory effect. The localized inflammatory response in the inner layer of the arterial wall has been identified as being responsible for many of the aspects of the greater thickness and rupture of the plaque, leading to acute cardiovascular episodes. The correlation between plasma C-reactive protein levels and cardiovascular pathologies has allowed investigators to use the levels of this protein as an excellent diagnostic tool together with total cholesterol levels, HDL ratios and homocysteine levels. The importance of hypercholesterolemia or, more specifically, a high low density (LDL) cholesterol concentration, as the main risk factor for the development of heart diseases, is widely accepted.

There are many types of synthetic drugs which are effective as hypocholesterolemic agents, however; the cost and the potential side effects of these drugs have led many people to search for natural cholesterol reducing substances.

In the last few years, the oxidative hypothesis of atherogenesis has opened up the path for therapies with antioxidant agents. Briefly, in situations in which low density lipoproteins (LDL and VLDL) are oxidized *in vivo*, foam cells are generated which finally lead to atherosclerosis. According to this hypothesis, the antioxidant should protect lipoproteins against the oxidative modification thereof and reduce its biological consequences. There is substantial evidence suggesting that the oxidative modifications of low density lipoproteins (LDL) critically contribute to the pathogenesis and progression of atherosclerosis in humans. Oxidized LDLs (oxLDLs) are present in atheromatous plaques and contain oxysterols showing a variety of adverse biological activities.

Polyphenolic compounds clearly show marked inhibitory effects on LDL oxidation. The presence of increasing concentrations of these compounds in the oxidation medium decreases LDL consumption through receptors. These polyphenols thus reduce the lipoprotein oxidation rate and inhibit atheromatous plaque growth.

The results show that antioxidants, mainly phenolic compounds, which are present in extra virgin olive oil and at a greater concentration in olive leaves, can contribute to the endogenous antioxidant capacity on LDLs, resulting in an increase of oxidation resistance as has been shown *in vitro.* Thus, the beneficial effects shown by olive oil on the inhibition of low density lipoprotein (LDL) oxidation depend not only on an increase in the intake of mono-unsaturated fatty acids (e.g. oleate) which are more stable to oxidation, but also on antioxidant phenols.

Many studies perform analytical determinations of the presence of phenolic compounds in a great variety of fruits and citrics (ANALYST THE: "Sample Preparation in the Determination of Phenolic Compounds in Druits", 2000, Published by the Roual Society of Chemistry, pages 1-26, XP008091061). Their role is shown by Del Rio, who teaches about the function of the phenolic compounds in olive and citrics within the plant, avoiding fungi infections (Del Rio, J.A. et al. "Involvement of phenolic compounds in the antifungal defense mechanisms of Olea europaea L. and Citrus sp", 2000, Recent Research Developments Ina Agricultural & Food Chemistry, Vol. 4, No. 2, pages 331-341).

The polyphenolic compounds present in virgin olive oil and in olive leaves also have anti-inflammatory and antioxidant effects in cardiovascular diseases. Tripoli makes a general revision of the activity of the polyphenol compounds present in olive oil (Tripoli E. et al. "The phenolic compounds of olive oil: structure, biological activity and beneficial effects on human health", 2005, Nutrition Research Reviews, Vol. 18, No. 1, pages 98-112).

The phenolic compounds present in olives can explain some of the protective effects found in epidemiological studies. Recent studies have shown a synergistic effect of olive phenolic compounds, the combination of which is much more potent in preventing the formation of oxidized cholesterol species and structural apoprotein modifications than the pure compounds individually, in the same way as with its antioxidant properties.

WO 97/32947 A1 teaches the combination of olive oil with a plant of the Citrus genus in an undefined and too general propose. The document does not describe the polyphenols present in Citrus, nor specify any active rate for a suitable combination of them. It teaches about olive oil but not about an extract of the olive plant, as the present invention does. The components of each, olive oil and plant, their activity and availability towards isolation are not similar, and results obtained at olive oil cannot be extrapolated to the isolation of a particular component of the plant. In particular, the present invention isolates oleuropein from the plant, whereas the concentration of oleuropein in olive oil is trivial. Consistently, this document does not teach by itself about the anticholesterolaemic effect of oleuropein in olive oil.

The addition of oleuropein in the diet increases the possibility of LDLs for resisting oxidation (less formation of conjugated dienes) and at the same time, for reducing the total, free and esterified plasma cholesterol levels (-15, -12, and -17%, respectively), giving rise to a redistribution of the LDL lipid components (larger amounts of phospholipids and cholesterol) with an indirect effect on their dimensions (about 12% higher.)

Hydroxytyrosol has shown the greatest protection capacity against the formation of oxidized phospholipids. The presence of oleuropein at low concentrations (5, 15 microM) does not prevent the oxidation of these phospholipids (8.02 +/- 1.22 and 1.22 +/- 1.22), but at concentrations higher than 30 microM it completely prevents the formation of this molecule, whereas hydroxytyrosol had no effect at any of the tested concentrations and allowed the oxidation of both phospholipids and cholesterol.

In addition, some bioflavonoids have been associated with the prevention of chronic diseases such as cancer and hyperlipidemia. Citrus fruits contain several bioflavonoids, flavanones, naringin and hesperidin being among the most important ones. These flavonoids have been pharmacologically evaluated as potential anticancer agents, anti-inflammatory agents and anti-clastogenic agents. The effects of these bioflavonoids on cholesterol metabolism were recently examined in rats fed with a cholesterol-rich diet.

### Description of the invention

Based on the foregoing, the present invention provides a functional food composition based on a combination of citrus and olive extracts with a Spanish origin, wherein the starting plant materials are subjected to a process in which they are intimately mixed and homogenized, before extracting the compounds. This process allows a co-crystallization of the active ingredients, which increases their water solubility and synergistically increases the anti-lipoperoxidant activity of oleuropeosides and flavonoids.

Said extracts are combined in the suitable proportions for increasing their bioavailability and their activity in the regulation of blood cholesterol and triglyceride levels. It is therefore a synergistic composition of different natural substances obtained by means of extraction from different citrus and olive species.

According to a first aspect, the food composition comprises the following phenolic compounds: between 5 and 25% oleuropein; between 1 and 5% neohesperidin; between 1 and 10% naringin; between 0.5 and 5% naringenin;between 0.1 and 2% apigenin and between 0.1 and 1% verbascoside and between 0.1 and 1% hydroxytyrosol. The percentages are expressed by weight.

The orange and lemon extracts can be obtained with several methods such as extraction with solvents, superheated water or supercritical CO₂.

The present invention uses a water-alcohol extraction significantly improving the water solubility of the compounds obtained and therefore their bioavailability.

According to a preferred embodiment, the composition comprises: between 14 and 16% oleuropein; between 0.1 and 1% hydroxytyrosol; between 0.1-1% verbascoside; between 5 and 6% naringin; between 1 and 2% naringenin; between 2 and 4% neohesperidin and between 0.4 and 1% apigenin.

Different doses of the composition developed herein are used and formulated in different functional foodstuffs and dietary supplements for preventing, reducing or treating hypercholesterolemia, hyperlipidemia and diseases derived therefrom such as cardiovascular diseases.

Suitable formulations of the present composition for its inclusion in functional foodstuffs and dietary supplements include liquid preparations such as solutions in glycerin/water or lecithin/water mixtures, or solid preparations such as micronization or microencapsulation. The recommended doses for supplementing the diet are between 700-800 mg/day, which is equivalent to about 350-400 mg/meal.

According to a second aspect, the invention relates to the use of the composition as an additional ingredient in food products such as dairy products, carbonated beverages, juices, bakery products and supplements.

### Brief Description of the Drawings

Figure 1 shows the effect of the composition of the invention on the total cholesterol, LDL, HDL and triglyceride blood level in volunteer groups A, B and C.
Figure 2 is a graph showing the relationship of the dose of the composition of the invention and blood cholesterol.

### Embodiment

The citrus and olive plant materials are mixed together and homogenized. The water-alcohol extraction is then carried out and subsequently, the citrus and olive extracts are combined to obtain a composition rich in phenolic compounds: 15% oleuropein; 0.5% hydroxytyrosol; 0.3% verbascoside; 5.5% naringin; 1.6% naringenin; 3% neohesperidin and 0.8% apigenin.

Effect of the daily intake of the composition of the invention on total cholesterol levels in several volunteers with high blood cholesterol levels (300-325 mg/dl).

### Volunteer groups

A. 6 volunteers (2 women and 4 men; 38-54 years) chronically treated with statins, who had suspended the statin treatment in May/June/2001, starting the treatment with the composition in September/2001.

B. 18 volunteers (10 women and 8 men; 48-76 years) with hyperlipidemia and treated discontinuously with statins. Start of the treatment with the composition in September/2001.

C. 12 volunteers (7 women and 5 men; 29-43 years) with hyperlipidemia, like group B, but without prior pharmacological treatment. Start of the treatment with the composition in September/2001.

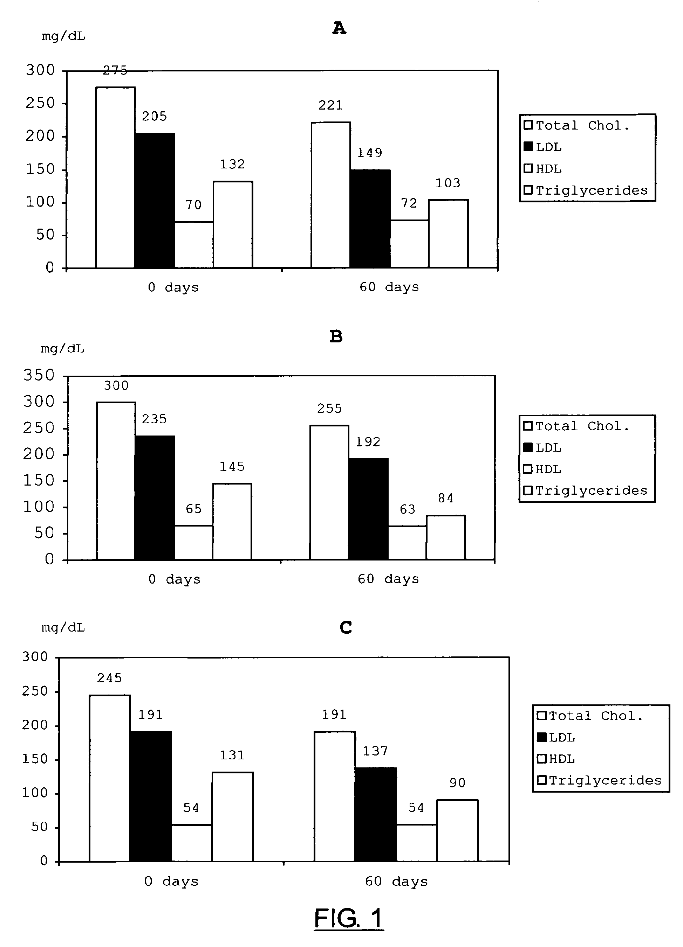

The composition was administered orally in doses of 800 mg/day in two gelatin capsules every 12 hours, for 60 days of treatment. The results obtained are shown in Table 1 and Figure 1.

**Table 1**

| Reduction of the atherogenic index (total cholesterol - HDL)/HDL) in Groups A, B and C, treated for 60 days with 800 mg/day of the composition. | | | |
|---|---|---|---|
| Volunteers | before | after | reduction (%) |
| Group A | 2.92 | 2.06 | 29.4 |
| Group B | 3.61 | 3.05 | 15.2 |
| Group C | 3.53 | 2.53 | 28.3 |

Figure 1 shows the average effect on the total cholesterol, LDL, HDL and triglyceride levels in the volunteers of Groups A, B and C treated for 60 days with 800 mg/day of the composition.

In order to obtain a dose-response curve, 3 volunteers of each experimental group were treated with different doses of the composition for 3 months.

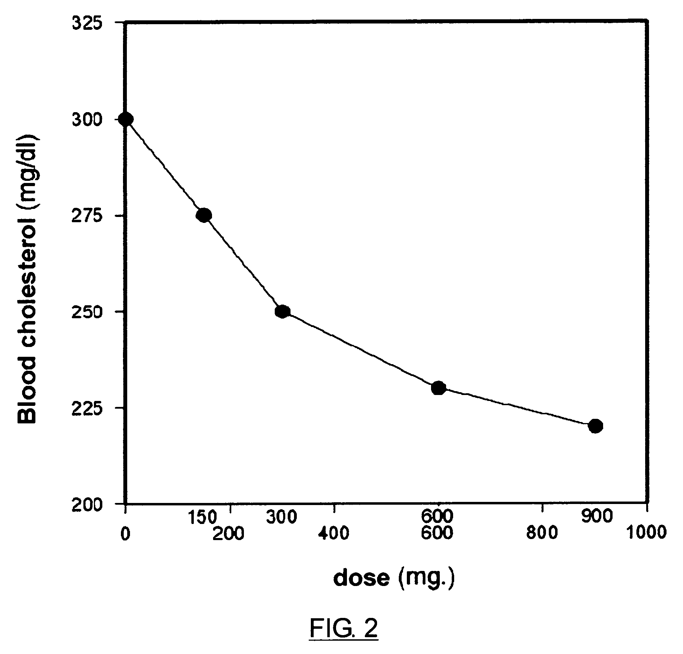

Figure 2 shows an asymptotic behavior in the reduction of blood cholesterol levels, suggesting that the latter decrease in a dose-dependent relationship with the ingestion of the composition and, furthermore, the existence of a regulatory effect on the distribution, oxidative processes, synthesis and/or exogenous absorption of cholesterol. This data is consistent with some prior results for citric flavonoids (Vilcox et al., 1999) and olive phenols (Visioli et al., 2000).

The results obtained with the treatments with the composition of the invention show a marked inhibitory effect on LDL oxidation. This combination not only improves the oxidation resistance of lipoproteins but also improves the lipid profile, combining the advantages of a lower cholesterol level and less LDL oxidation.

The decrease in LDL levels is very significant, approximately 20%. This effect would be due to less triglyceride synthesis which would reduce the formation of VLDL by hepatocytes and subsequently the formation of their metabolite, LDL.

The composition combines the advantages of maintaining low cholesterol and triglyceride levels and less LDL oxidation with the analgesic and anti-inflammatory properties inherent to its components and linked to the inhibition of arachidonic acid metabolism.

## Claims

1. A functional food composition obtained from the combination of citric and olive species extracts, comprising the following phenolic compounds: between 5 and 25% of oleuropein; between 0.1 and 1% of verbascoside; between 0.1 and 1% of hydroxytyrosol, between 1 and 5% of neohesperidin; between 1 and 10%, of naringin; between 0.5 and 5% of naringenin and between 0.1 and 2% of apigenin.

2. A composition according to claim 1, **characterized in that** said composition comprises between 14 and 16% by weight of oleuropein.

3. A composition according to claim 1, **characterized in that** said composition comprises between 5 and 6%, by weight of naringin.

4. A composition according to claim 1, **characterized in that** said composition comprises between 1 and 2% by weight of naringenin.

5. A composition according to claim 1, **characterized in that** said composition comprises between 2 and 4% by weight of neohesperidin.

6. A composition according to claim 1, **characterized in that** said composition comprises between 0.4 and 1%, by weight of apigenin.

7. Use of the composition according to claim 1, **characterized in that** said composition is added as an ingredient in food products selected from carbonated beverages, juices and bakery products.

8. Composition according to any one of claims 1 to 6, **characterized in that** said composition is a dietary supplement for use in preventing, reducing or treating hypercholesterolemia, hyperlipidemia and cardiovascular diseases.

9. A composition for use according to claim 8, **characterized in that** said dietary supplement is a liquid preparation.

10. A composition for use according to claim 9, **characterized in that** said liquid preparation is a solution in glycerin/water or lecithin/water mixtures.

11. A composition for use according to claim 8, **characterized in that** said dietary supplement is a solid preparation.

12. A composition for use according to claim 11, **characterized in that** said solid preparation is a micronization or a microencapsulation.

13. The composition according to claim 1 for use in the inhibition of atherosclerosis.

## Patentansprüche

1. Functional Food-Zusammensetzung, erhalten aus der Kombination von Extrakten aus Zitrus- und Olivenarten und umfassend die folgenden phenolischen Verbindungen: zwischen 5 und 25% Oleuropein; zwischen 0,1 und 1% Verbascosid; zwischen 0,1 und 1% Hydroxytyrosol, zwischen 1 und 5% Neohesperidin; zwischen 1 und 10% Naringin; zwischen 0,5 und 5% Naringenin und zwischen 0,1 und 2% Apigenin.

2. Zusammensetzung nach Anspruch 1, **dadurch gekennzeichnet, dass** die genannte Zusammensetzung zwischen 14 und 16 Gew.-% Oleuropein umfasst.

3. Zusammensetzung nach Anspruch 1, **dadurch gekennzeichnet, dass** die genannte Zusammensetzung zwischen 5 und 6 Gew.-% Naringin umfasst.

4. Zusammensetzung nach Anspruch 1, **dadurch gekennzeichnet, dass** die genannte Zusammensetzung zwischen 1 und 2 Gew.-% Naringenin umfasst.

5. Zusammensetzung nach Anspruch 1, **dadurch gekennzeichnet, dass** die genannte Zusammensetzung zwischen 2 und 4 Gew.-% Neohesperidin umfasst.

6. Zusammensetzung nach Anspruch 1, **dadurch gekennzeichnet, dass** die genannte Zusammensetzung zwischen 0,4 und 1 Gew.-% Apigenin umfasst.

7. Verwendung der Zusammensetzung nach Anspruch 1, **dadurch gekennzeichnet, dass** die genannte Zusammensetzung als Zutat in Lebensmitteln, ausgewählt aus kohlensäurehaltigen Getränken, Säften und Backwaren, hinzugefügt wird.

8. Zusammensetzung nach einem der Ansprüche 1 bis 6, **dadurch gekennzeichnet, dass** die genannte Zusammensetzung ein Nahrungsergänzungsmittel für dessen Verwendung bei der Vorbeugung, Verminderung oder Behandlung von Hypercholesterinämie, Hyperlipidämie und Herz-Kreislauferkrankungen, ist.

9. Zusammensetzung für deren Verwendung nach Anspruch 8, **dadurch gekennzeichnet, dass** das genannte Nahrungsergänzungsmittel eine flüssige Zubereitung ist.

10. Zusammensetzung für deren Verwendung nach Anspruch 9, **dadurch gekennzeichnet, dass** die genannte flüssige Zubereitung eine Lösung in Glycerin/Wasser- oder Lecithin/Wasser-Mischungen ist.

11. Zusammensetzung für deren Verwendung nach Anspruch 8, **dadurch gekennzeichnet, dass** das genannte Nahrungsergänzungsmittel eine feste Zubereitung ist.

12. Zusammensetzung für deren Verwendung nach Anspruch 11, **dadurch gekennzeichnet, dass** die genannte feste Zubereitung eine Mikronisierung oder eine Mikroverkapselung ist.

13. Zusammensetzung nach Anspruch 1 für deren Verwendung bei der Hemmung von Atherosklerose.

## Revendications

1. Composition alimentaire fonctionnelle obtenue à partir de la combinaison d'extraits d'espèces citriques et d'olives, comprenant les composants phénoliques suivant: entre 5 et 25% d'oleuropéine; entre 0,1 et 1% de verbascoside; entre 0,1 et 1% d'hydroxytyrosol; entre 1 et 5% de néohespéridine; entre 1 et 10% de naringine; entre 0,5 et 5% de naringénine et entre 0,1 et 2% d'apigénine.

2. Composition selon la revendication 1, **caractérisée en ce que** ladite composition comprend entre 14 et 16% en poids d'oleuropéine.

3. Composition selon la revendication 1, **caractérisée en ce que** ladite composition comprend entre 5 et 6% en poids de naringine.

4. Composition selon la revendication 1, **caractérisée en ce que** ladite composition comprend entre 1 et 2% en poids de naringénine.

5. Composition selon la revendication 1, **caractérisée en ce que** ladite composition comprend entre 2 et 4% en poids de néohespéridine.

6. Composition selon la revendication 1, **caractérisée en ce que** ladite composition comprend entre 0,4 et 1% en poids d'apigénine.

7. Utilisation de la composition selon la revendication 1, **caractérisée en ce que** ladite composition est ajoutée comme un ingrédient dans des produits alimentaires choisis parmi les boissons gazeuses, les jus et les produits de boulangerie.

8. Composition selon l'une des revendications 1 à 6, **caractérisée en ce que** ladite composition est un supplément diététique pour l'utilisation dans la prévention, la réduction ou le traitement de l'hypercholestérolémie, de l'hyperlipidémie et de maladies cardiovasculaires.

9. Composition pour l'utilisation selon la revendication 8, **caractérisée en ce que** ledit supplément diététique est une préparation liquide.

10. Composition pour l'utilisation selon la revendication 9, **caractérisée en ce que** ladite préparation liquide est une solution dans des mélanges glycérine/eau ou lécithine/eau.

11. Composition pour l'utilisation selon la revendication 8, **caractérisée en ce que** ledit supplément diététique est une préparation solide.

12. Composition pour l'utilisation selon la revendication 11, **caractérisée en ce que** ladite préparation solide est une micronisation ou une microencapsulation.

13. Composition selon la revendication 1, utilisée pour l'inhibition de l'athérosclérose.
